# EUROPEAN PATENT SPECIFICATION

(11) **EP 0 341 962 B1**
(45) Date of publication and mention of the grant of the patent: **15.03.1995**
(21) Application number: 89304646.6
(22) Date of filing: 08.05.1989
(51) Int. Cl.: C07K 14/00, A61K 38/04

(54) **Humoral hypercalcemic factor antagonists**
Hemmer vom flüssigen kalziumerhöhenden Faktor
Antagonistes du facteur humoral hypercalcémique

(30) Priority: 09.05.1988 US 191513; 09.05.1988 US 191514
(43) Date of publication of application: 15.11.1989
(73) Proprietor: Merck & Co., Inc., Rahway New Jersey 07065-0900 (US)
(72) Inventor: Rosenblatt, Michael, Ardmore, PA 19003 (US); Caulfield, Michael P., Lansdale, PA 19446 (US); McKee, Roberta L., Lansdale, PA 19446 (US); Nutt, Ruth F., Green Lane, PA 18054 (US)
(74) Representative: Cole, William Gwyn

(56) References cited:
- WO-A-88/00596
- US-A- 4 423 037
- J. ENDOCRINOLOGY, vol. 108, no. 2, 1986, pages 261-265, Journal OfEndocrinology Ltd, GB; M. KUBOTA et al.: "Efficacy and specificity of humanparathyroid hormone analogues as antagonists in intact clonal osteogenicsarcoma cells"
- THE NEW ENGLAND JOURNAL OF MEDICINE, vol. 315, no. 16, 1986, pages 1004-1013;M. ROSENBLATT: "Peptide hormone antagonists that are effective in vivo"
- PEPTIDES, CHEMISTRY AND BIOLOGY, PROCEEDINGS OF THE TENTH AMERICAN PEPTIDESYMPOSIUM, St. Louis, Missouri, 23rd-28th May 1987, pages 449-451, ESCOMScience Publishers B.V., Leiden, NL; L.H. CAPORALE et al.: "Characterization ofparathyroid hormone antagonists"
- SCIENCE, vol. 238, 1987, pages 1566-1568; N. HORIUCHI et al.: "Similarity ofsynthetic peptide from human tumor to parathyroid hormone in vivo and in vitro"
- BIOCHEMICAL AND BIOPHYSICAL RESEARCH COMMUNICATIONS, vol. 158, no. 3, 15thFebruary 1989, pages 1036-1042, Academic Press, Inc.; K. NAGASAKI et al.: "Invitro and in vivo antagonists against parathyroid hormone-related protein"
- ENDOCRINOLOGY, vol. 122, no. 6, 1988, pages 3008-3010; R.L. McKEE et al.: "The7-34-fragment of human hypercalcemia factor is a partial agonist/antagonist forparathyroid hormone-stimulated cAMP production"

## Description

### BACKGROUND OF THE INVENTION

This invention relates to the use of peptide analogues useful in inhibiting the naturally occurring peptide in vivo and in vitro. These peptide analogues when administered to a vertebrate, such as mammals, block the activity of the peptide or other analogous molecules. These peptide analogues are also useful in vitro in combination with a bioassay for the naturally occurring peptide. The peptide analogues are useful in treating various diseases caused by excess of the naturally occurring peptide and in treating peptide dependent tumors. One example of this invention relates to the use and synthesis of humoral hypercalcemic factor (HCF) analogues useful for inhibiting the action of HCF both in vivo and in vitro.

Recently, several investigators isolated and obtained partial amino acid sequences of peptide derived from several different human tumors (lung squamous carcinoma, renal cell carcinoma, and breast carcinoma). J.M. Moseley et al., Proc. Natl. Acad. Sci. U.S.A. 84, 5048 (1987); G.J. Strewler et al., J. Clin. Invest., 80, 1803 (1987); A.F. Stewart et al., Biochem. Biophys. Res. Commun. 146, 672 (1987); M. Mangin et. al., Proc. Natl. Acad. Sci. U.S.A., 85, 597 (1988). One group published the putative full-length peptide structure (141 amino acids) based on the complementary DNA (cDNA) nucleotide sequence. L.J. Suva et al., Science 237, 893 (1987).

This new factor has been named human "humoral hypercalcemic factor" (hHCF) and is considered to be related in biological effects to parathyroid hormone (PTH). HCF shows considerable homology to the biologically critical NH₂-terminal region of PTH. However, there are significant differences in the peptide sequences between PTH and HCF, and this new factor appears to be the product of a different gene.

Previously, it had been proposed that tumors could secrete PTH ectopically and cause hypercalcemia of malignancy. However, messenger RNA for PTH was not found in such tumors. Several studies demonstrated that a PTH-like factor, physicochemically and immunologically distinct from PTH, is secreted by tumor cells. S.B. Rodan et al. J. Clin. Invest. 72, 1511 (1983); A.F. Stewart et al., Proc. Natl. Acad. Sci. U.S.A., 80, 1454 (1983); G.J. Strewler et al., J. Clin. Invest. 71, 769 (1983). It was also known that this PTH-like factor stimulates adenylate cyclase in PTH target cells, and that this activity can be inhibited by PTH antagonists. Thus, it is presently considered that HCF is a factor that is responsible for hypercalcemia of malignancy by its secretion from the tumor and its altering effect on calcium metabolism.

It is, therefore, an object of the present invention to provide antagonists of HCF. If a peptide analogue of HCF could be constructed which would bind with the cell surface receptor of HCF, then the peptide analogue could be used to block the effect of the naturally occurring peptide. Thus, it is also an object of the present invention to provide peptide analogues useful for the treatment of hypercalcemia of malignancy.

Another object of the present invention is to provide novel HCF analogues. Other objects of the present invention are to provide methods of inhibiting the action of HCF through the administration of novel HCF analogues. Still another object of the invention is to provide HCF analogues wherein amino acid modifications result in binding to the cell surface receptors without activating a second messenger molecule. The above and other objects are accomplished by the present invention in the manner more fully described below.

### SUMMARY OF THE INVENTION

The present invention provides, in a first part, peptides selected from the group consisting of hHCF(14-34)NH₂, hHCF(13-34)NH₂, hHCF(12-34)NH₂, hHCF(11-34)NH₂, hHCF(10-34)NH₂, hHCF(9-34)NH₂, and hHCF(8-34)NH₂.

Additionally, the first part of present invention provides the above peptides wherein (a) Ala³⁴ is replaced with Tyr³⁴, and/or (b) Phe²³ is replaced with a hydrophobic amino acid selected from the group consisting of the D- or L-stereoisomers of Leu, Nle, Val, Tyr, Trp, beta-naphthylalanine and alpha-naphthylalanine, and/or (c) any or each of Asp¹⁰, Lys¹¹ or Ile¹⁴ is replaced with an N-alkyl amino acid, or a D- or L-stereoisomer of an amino acid, in particular Asn, Leu or His.

The peptides selected from the group consisting of hHCF(12-34)NH₂_{,} hHCF(11-34)NH₂, hHCF(10-34)NH₂, hHCF(9-34)NH₂ and hHCF(8-34)NH₂ wherein Gly¹² is replaced with an amino acid selected from the group consisting of the D- or L-stereoisomers of Trp, Pro, Ala, Aib, naphthyl Ala, alpha-MeTrp and NMe Gly are also an aspect of the first part of present invention. These first parts peptides wherein (a) Ala³⁴ is replaced with Tyr³⁴, and/or (b) Phe²³ is replaced with a hydrophobic amino acid selected from the group consisting of the D- or L-stereoisomers of Leu, Nle, Val, Tyr, Trp, beta-naphthylalanine and alpha-naphthylalanine, and/or (c) any or each of Asp¹⁰, Lys¹¹ or Ile¹⁴ is replaced with an N-alkyl amino acid or a D- or L-stereoisomer of an amino acid, in particular Asn, Leu or His, are also included.

Another feature of the first part of present invention is the peptide which is [Nle⁸,¹⁸] hHCF (8-34) NH₂ wherein (a) Ala³⁴ is replaced with Tyr³⁴, and/or (b) Phe²³ is replaced with an amino acid selected from the group consisting of the D- or L-stereoisomers of Leu, Nle, Val, Tyr, Trp, beta-naphthylalanine and alpha-naphthylalanine, and/or (c) Gly¹² is replaced with an amino acid selected from the group consisting of the D- or L- stereoisomers of Trp, Pro, Ala, Aib , naphthyl Ala, alpha-MeTrp and NMe Gly, and/or (d) any or each of Asp¹⁰, Lys¹¹ or Ile¹⁴ is replaced with an N-alkyl amino acid or a D- or L-stereoisomer of an amino acid, in particular Asn, Leu or His. A preferred peptide is hHCF(14-34)NH₂, and the peptide containing the substitutions indicated above where permissible.

In a second part, the present invention provides peptides selected from the group consisting
of hHCF(3-34)NH₂,
[Tyr³⁴]hHCF(3-34)NH₂, [Nle^{8,18},
Tyr³⁴]hHCF(3-34)NH₂, [Nle^{8,18}]hHCF(3-34)NH₂;
hHCF(4-34)NH₂; [Tyr³⁴]hHCF(4-34)NH₂,
[Nle^{8,18}, Tyr³⁴]hHCF(4-34)NH₂,
[Nle^{8,18}]hHCF(4-34)NH₂; hHCF(5-34)NH₂,
[Tyr³⁴]hHCF(5-34)NH₂, [Nle^{8,18},
Tyr³⁴]hHCF(5-34)NH₂, [Nle^{8,18}]hHCF(5-34)NH₂;
hHCF(6-34)NH₂, [Tyr³⁴]hHCF(6-34)NH₂,
[Nle^{8,18}, Tyr³⁴]hHCF(6-34)NH₂,
[Nle^{8,18}]hHCF(6-34)NH₂; hHCF(7-34)NH₂,
[Tyr³⁴]hHCF(7-34)NH₂, [Nle^{8,18}
Tyr³⁴]hHCF(7-34)NH₂, and [Nle^{8,18}]hHCF(7-34)NH₂.

Additionally, the second part of the present invention provides the above peptides wherein (a) Gly¹² is replaced with an amino acid selected from the group consisting of the L- or D-stereoisomers of Trp, Pro, Ala, Aib, naphthyl Ala, alpha-MeTrp and NMe Gly, and/or (b) any or each of Asp¹⁰, Lys¹¹ or Ile¹⁴ is replaced with any N-alkyl containing, or D- or L- stereoisomer of, any amino acid, in particular Asn, Leu or His.

Preferred peptides of the second part are
[D-Trp¹²]hHCF(7-34)NH₂, [D-Trp¹²,
Tyr³⁴]hHCF(7-34)NH₂, [D-Trp¹²,Nle^{8,18},
Tyr³⁴]hHCF(7-34)NH₂, [D-Trp¹², Nle
^{8,18}]hHCF(7-34)NH₂, [Asn¹⁰,
Leu¹¹]hHCF(7-34)NH₂, [Asn¹⁰, Leu¹¹,
Nle^{8,18}]hHCF(7-34)NH₂, [Nle^{8,18}]hHCF
(7-34)NH₂, [Asn¹⁰,Leu¹¹, Nle^{8,18},
Tyr³⁴]hHCF(7-34)NH₂, [Asn¹⁰, Leu¹¹,
Tyr³⁴]hHCF(7-34)NH₂, [Nle^{8,18},
Tyr³⁴]hHCF(7-34)NH₂.

Also preferred are hHCF(7-34)NH₂,
[Asn¹⁰, D-Trp¹²]hHCF(7-34)NH₂, [Leu¹¹
D-Trp¹²]hHCF(7-34)NH₂,[Ser¹⁰, Leu¹¹,
D-Trp¹²]hHCF(7-34)NH₂, [Ser¹⁰, Pro¹¹,
D-Trp¹²]hHCF(7-34)NH₂, [Ser¹⁰, Sar¹¹,
D-Trp¹²]hHCF(7-34)NH₂, [Leu¹¹, D-Trp¹²,
Tyr³⁴]hHCF(7-34)NH₂.

Use of the terms "and/or" in the above description of the invention means that the substitutions described can be made singly or in any and all combinations described. For example, each of hHCF(14-34)NH₂,[Tyr³⁴]hHCF(14-34)NH₂, [Leu²³] hHCF(7-34)NH₂, [Leu²³,Tyr³⁴]hHCF(14-34)NH₂, [Leu¹¹]hHCF(7-34)NH₂, [Leu^{11,23}]hHCF(7-34)
NH₂, [Leu ^{11,23},Tyr³⁴]hHCF(7-34)NH₂, as well as the other described combinations, are included within the present invention.

Any of the above-mentioned peptides can be used in a method of acting upon a HCF receptor which comprises administering an effective amount of such peptide to a mammal. Additionally, an in vitro bioassay of HCF, wherein a measured amount of such peptides inhibits binding of HCF to a HCF receptor in vitro is an aspect of the present invention. A pharmaceutical composition which comprises an effective amount of any such a peptide and a pharmaceutically acceptable carrier is another feature of this invention.

The present invention provides a method of inhibiting the action of HCF comprising the administration of therapeutically effective amount of HCF analogues described above. The present invention also provides a method of treating osteoporosis or hypercalcemia comprising the administration of a therapeutically effective amount of a HCF analogue described above. A method of treating hyperparathyroidism comprising the administration of a therapeutically effective amount of the HCF analogues of this invention is also provided. A method of treating hyperparathyroidism expressed as a hypercalcemic crisis, renal failure or hypertension is also provided. A method of treating the disease state produced by a tumor or other cell overproducing a peptide hormone-like molecule and method of treating immune diseases wherein the disease state comprises inflammation, an allergic response, or hyperactive lymphocytes is also provided by the novel peptide analogues of the present invention.

### DETAILED DESCRIPTION OF THE INVENTION

Various other objects, features and attendent advantages of the present invention will be more fully appreciated as the same becomes better understood from the following detailed description.

Extensive structure and activity studies have now led to the design of peptide analogues which have high binding affinity for their respective cell surface receptors while not stimulating the production of second messenger molecules.

HCF analogues with two to thirteen amino acids removed from the N-terminus produces an inhibitor which still binds with high affinity to the peptide hormone receptor without causing a change in cyclic AMP concentration.

The following is the 34-amino acid sequence of human humoral hypercalcemia factor (hHCF):
Ala-Val-Ser-Glu-His(5)-Gln-Leu-Leu-His-Asp(10)-Lys-Gly-Lys-Ser-Ile(15)-Gln-Asp-Leu-Arg-Arg-(20)-Arg-Phe-Phe-Leu-His(25)-His-Leu-Ile-Ala-Gln(30)-Ile-His-Thr-Ala. Standard abbreviations well recognized in the peptide chemistry art are utilized herein. Aib represents an aminoisobutyrl substitutent.

Fragments of peptide, containing the region specific for binding to the cell surface receptor can be used as inhibitors or blocking agents. For HCF, it is considered that the N-terminal 34 amino acids are sufficient to define binding specificity to the cell surface receptor.

The presence of D-amino acids in peptide in place of L-amino acids sometimes results in a peptide resistant to catabolism. However, not all such substitutions result in an active peptide. Thus, such substitutions which result in active peptide are considered to be within the scope of the present invention. The utilization of D-amino acids in peptide hormone synthesis is described in the following publications herein incorporated by reference: Coltrera, et al., Biochemistry, 19:4380-4385, 1980; Rosenblatt et al., Biochemistry, 20:7246-7250, 1981. Additionally, substitutions of amino acids which are equivalent to the amino acids disclosed herein is considered to be within the scope of the present invention.

The balance of the description will be divided into two sections. Section I will describe the preparation and structure of inhibitors of peptide hormones, Section II will discuss the use of the peptide hormone inhibitors.

### I. Preparation and Structure of Peptide Hormone Inhibitors

The technique of solid-phase peptide synthesis, developed by Merrifield ("Solid-Phase Peptide Synthesis", Advances in Enzymology, 32:221-296, (1969); G. Barany and R.B. Merrifield "Solid-Phase Peptide Synthesis" in The Peptides, volume 2, editors: E. Gross & J. Meienhofer (1980)) has been successfully employed in the synthesis of peptides including HCF. This method is based on the strategy of having the carboxyl terminus of the peptide linked covalently to a solid support. The desired peptide sequence is prepared by stepwise coupling of single amino acids to a peptide chain growing from the carboxyl toward the amino terminus. Coupling is typically achieved by activation of the carboxyl group of the amino acid being attached to the resin, which may have other potentially reactive groups blocked. Following addition of an amino acid to the growing polypeptide chain, and prior to further chain elongation, the alpha-amino (Boc) protecting group is typically removed. Because each amino acid is coupled by nearly the same series of reactions, the need for elaborate strategies in the synthesis is minimized. Solublility is not a major issue during synthesis, because the peptide is linked to a solid support. This method is rapid and it can be utilized by a single worker. It is very convenient for the synthesis of multiple analogues with amino-terminal substitutions, because a single synthesis can be branched in multiple directions near the amino terminus, thereby creating many analogues varying only in the amino terminal region.

### II. Use of Peptide Inhibitors

The method of inhibiting the action of HCF peptide comprises the administration of a therapeutically effective amount of any HCF peptide analogue. These peptide analogues retain specificity for the cell surface receptor without stimulating a physiological response. This method of use applies to the entire peptide or its analogue, or to a fragment of the peptide or analogue containing the receptor binding site.

The use of peptide analogues is exemplified by HCF analogues. The HCF is of human origin but HCF of bovine, rat or any mammalian source may prove to be equivalent to the human HCF. The analogue may contain all the amino acids indicated, or additionally truncations or elongations. Individual amino acids can be substituted to improve biological or chemical stability.

The peptide analogues of this invention can be used in vitro to measure the concentration of naturally occurring peptide. This bioassay procedure is illustrated by a bioassay for HCF. The unknown concentration of HCF in a solution can be determined by measuring the amount of HCF analogue required to inhibit its binding to the HCF cell surface receptor. The concentration of HCF analogue required to block the action of HCF is a direct indicator of the HCF concentration.

HCF analogues can be used to diagnose the etiology of or to treat osteoporosis or hypercalcemia through the administration of a therapeutically effective amount of the HCF analogues of this invention. Similarly, hyperparathyroidism and other aspects of hyperparathyroidism, such as a hypercalcemic crisis, renal failure or hypertension can be treated through the administration of the HCF analogues of this invention.

Tumors and other aberrant cell growth often produce hormone-like substances causing a disease state. The use of peptide analogues to block stimulation caused by such hormone like substances can result in the alleviation of the disease state. An example of this is the humoral hypercalcemic factor of malignancy. Therefore, the HCF peptide analogues of the present invention can be administered to treat diseases caused by aberrant production of hormone-like substances.

The peptide analogues of this invention exhibit both oral and parenteral activity and can be formulated in dosage forms for oral, parenteral, intra-nasal, or topical administration. Solid dosage forms for oral administration include capsules, tablets, pills, powders and granules. In such solid dosage forms, the active compound is admixed with at least one inert diluent such as sucrose, lactose or starch. Such dosage forms can also comprise, as is normal practice, additional substances other than inert diluent. In the case of capsules, tablets, and pills, the dosage forms may also comprise buffering agents. Tablets and pills can additionally be prepared with an enteric coating.

Liquid dosage forms for oral administration include pharmaceutically acceptable emulsion, solutions, suspensions, syrups and elixers containing inert diluents commonly used in the pharmaceutical art. Besides inert diluents, such compositions can also include adjuvants, such as wetting agents, emulsifying and suspending agents, and sweetening. Preparations according to this invention for parenteral administration include sterile aqueous or non-aqueous solutions, suspensions or emulsions. Examples of non-aqueous solvents or vehicles are propylene glycol, polyethylene glycol, vegetable oils such as olive oil and injectable organic esters such as ethyloleate.

The dosage of active ingredient in the compositions of this invention may be varied; however it is necessary the amount of the active ingredient shall be such that a suitable dosage form is obtained. The selected dosage form depends upon the desired therapeutic effect, on the route of the administration, and on the duration of the treatment.

Obviously, numerous modifications and variations of the present invention are possible in light of the above teachings. It is therefore to be understood that within the scope of the appended claims, the invention may be practiced otherwise than as specifically described herein.

### EXAMPLE 1

### Synthesis and Purification of Peptide Analogues of HCF

Analogues of HCF, where prepared by a modification of the solid-phase method of Merrifield. Syntheses were performed using an Applied Biosystems 430A Synthesizer. 4-Methyl-benzhydrylamine hydrochloride resin (polystyrene-1% by divinylbenzene, USB) was employed as the solid support in order to effect the carboxyamide (CONH2) COOH-terminal modification.

The tertiary butyloxycarbonyl (Boc) group was used to protect the alpha -amino group of each amino acid during coupling. Side-function protection was afforded as follows: (a) the hydroxyl group of serine was protected as the O-benzyl ether (Bzl); (b) the hydroxyl group of tryosine as the 0-2,6-dichlorobenzyl ether (DCB) or p-bromobenzyloxycarbonyl ester (Brz); (c) the carboxyl group of glutamic and aspartic acid as the benzyl (BZ) or cyclohexyl ester (Chx); (d) the imidazole nitrogen of histidine by the benzyloxymethyl (BOM) and the guanidine function of arginine was protected by the p-toluene-sulfonyl (TOS) group, and the indole imine by formyl groups (For); and (e) the Lysine epsilon amino group by 2-chloro-benzyloxycarboxyl (ClZ). All amino acids were obtained from Applied Biosystems, Inc., Peninsula Laboratories, or Bachem Chemicals.

The peptide-resin synthesis were carried out using Applied Biosystems, Inc. specified protocols. Double couplings were carried out for the incorporation of each amino acid. After the final coupling of each of the arginines (residues 18-21) the remaining free amino groups were acetylated to prevent generation of deletion peptides. Deprotection times with trifluoroacetic acid (TFA) were extended 6 minutes over manufacturer protocols.

The peptide was cleaved from the copolymer resin with simultaneous removal of the side-chain protecting groups similar to the 2 step HF cleavage procedure described by Tam, J.A.C.S. 105: 6442-6455 (1983). In the first HF step the following ratios of reagents were used: 5% p-cresol, 5% p-thiocresol, 65% dimethyl sulfide and 25% HF. 10 ml of mixture per gram of peptide-resin was used for 2 hours at 0°C. In the second HF step the following ratio of reagents were used: 5% p-cresol, 5% p-thiocresol and 90% HF. The cleavage was carried out for 75 min. at 0°C. After removal of HF the peptide-resin mixture was washed with anhydrous ether to remove scavenger. The peptide was then extracted with 50% acetic acid and water. The washes were combined and chromatographed using Sephadex G-50F, eluting with 50% HOAc.

After lyophilization, the partially purified peptide was chromatographed by reverse phase HPLC (Vydac C₄ bonded silica, 15 u particle size, 300A pore size, using aqueous acetonitrile gradient containing 0.1%TFA).

### EXAMPLE 2

### HCF Binding Assay Results

HCF analogues were analysed in a new receptor assay (Goldman et al., Emdrpcrinol (1988) 123: 1468-1475) which modified the assay reported in Rosenblatt et al., Endocrin. 107: 545-550 (1980). The binding assay used [Nle^{8,18}(¹²⁵I)-Tyr³⁴]bPTH (1-34)NH₂ which was purified by HPLC (Novapak C₁₈, 32-35% CH₃CN in 0.1% TFA) and was stored as aliquots in 25 mM TrisHCl/1%BSA at -70°C. Bovine renal cortical plasma membranes were incubated with radioligand (25,000 cpm) in the absence or presence of HCF analogs in a Tris-containing buffer (250 ul) for 30 min. at 21°C. Once equilibrium was reached, bound and free radioligand were separated by centrifugation. High specific binding (85%) to bovine renal cortical membranes was obtained consistently.

| Structure | Binding K_{I}(nM) |
|---|---|
| hHCF(14-34)NH₂ | 1400 ± 83 |
| [Asn¹⁰,Leu¹¹]hHCF(7-34)NH₂ | 104 ± 27 |
| hHCF(7-34)NH₂ | 257 ± 37 |
| [D-Trp¹²]hHCF(7-34)NH₂ | 50 ± 14 |
| [Asn¹⁰,D-Trp¹²]hHCF(7-34)NH₂ | 39 ± 15 |
| [Leu¹¹,D-Trp¹²]hHCF(7-34)NH₂ | 4 ± 2 |
| [Asn¹⁰,Leu¹¹,D-Trp¹²]hHCF(7-34)NH₂ | 7 ± 2 |
| [Ser¹⁰,Leu¹¹,D-Trp¹²]hHCF(7-34)NH₂ | 39 ± 15 |
| [Ser¹⁰,Pro¹¹,D-Trp¹²]hHCF(7-34)NH₂ | 113 ± 38 |
| [Ser¹⁰,SAR¹¹,D-Trp¹²]hHCF(7-34)NH₂ | 14 ± 5 |
| [D-Trp¹²]hHCF(10-34)NH₂ | 403 ± 251 |

### EXAMPLE 3

HCF analogues were analyzed in a bovine renal membrane adenylate cyclase assay as described in Horiuchi et al., Science 238, 1566 (1987); Goldman et al., Endocrin (1988) 123(5): 1468-1475. 3nM [Nle^{8,18,}Tyr³⁴]bPTH(1-34) NH₂ was used to stimulate adenylate cyclase.

| Structure | Binding K_{I}(nM) |
|---|---|
| hHCF(14-34)NH₂ | 6,600 ± 1300 |
| [D-Trp¹²]hHCF(10-34)NH₂ | 2,365 ± 650 |
| hHCF(7-34)NH₂ | 609 ± 133 |
| [D-Trp¹²]hHCF(7-34)NH₂ | 390 ± 183 |
| [Asn¹⁰,D-Trp¹²]hHCF(7-34)NH₂ | 244 ± 87 |
| [Leu¹¹,D-Trp¹²]hHCF(7-34)NH₂ | 33 |
| [Asn¹⁰,Leu¹¹,D-Trp¹²]hHCF(7-34)NH₂ | 73 ± 16 |
| [Ser¹⁰,Leu¹¹,D-Trp¹²]hHCF(7-34)NH₂ | 244 ± 87 |
| [Ser¹⁰,Pro¹¹,D-Trp¹²]hHCF(7-34)NH₂ | 1093 ± 430 |

### EXAMPLE 4

HCF analogues were analyzed in a rat osteosarcoma cell line, ROS 17/2.8, for the ability to inhibit cAMP stimulation by 1nM [Nle^{8,18},Tyr³⁴]bPTH(1-34) NH₂ by the method described by McKee, R. et al, Endocrinol. (1988) 122(6): 3008-10.

| Structure | Binding K_{I}(nM) |
|---|---|
| hHCF(14-34)NH₂ | 2040 ± 160 |
| hHCF(7-34)NH₂ | 90 ± 220 |
| [Asn¹⁰,Leu¹¹]hHCF(7-34)NH₂ | 37 ± 2 |
| [D-Trp¹²]hHCF(7-34)NH₂ | 132 ± 12 |
| [Asn¹⁰,D-Trp¹²]hHCF(7-34)NH₂ | 74 ± 9 |
| [Leu¹¹,D-Trp¹²]hHCF(7-34)NH₂ | 6.6 ± 0.9 |
| [Asn¹⁰,Leu¹¹,D-Trp¹²]hHCF(7-34)NH₂ | 8.9 ± 1.5 |
| [Ser¹⁰,Leu¹¹,D-Trp¹²]hHCF(7-34)NH₂ | 8.6 ± 2.7 |
| [Ser¹⁰,Pro¹¹,D-Trp¹²]hHCF(7-34)NH₂ | 51 ± 5 |

### Example 5

HCF analogues were assayed for their ability to inhibit PTH-stimulated erdenylate cyclose in human osteosarcoma cells (B10) in a similar manner to that described in Example 4.

| Structure | Binding K_{I}(nM) |
|---|---|
| hHCF(7-34)NH₂ | 77 ± 11 |
| [Asn¹⁰,Leu¹¹]hHCF(7-34)NH₂ | 9 |
| [D-Trp¹²]hHCF(7-34)NH₂ | 17 ± 6 |
| [Asn¹⁰,D-Trp¹²]hHCF(7-34)NH₂ | 3.5 |
| [Leu¹¹,D-Trp¹²]hHCF(7-34)NH₂ | 2.2 ± 0.4 |
| [Leu¹¹,D-Trp¹²Tyr³⁴]) | 5.9 ± 0.9 |
| hHCF(7-34)NH₂ | |
| [Asn¹⁰,Leu¹¹,D-Trp¹²] | 3.5 ± 0.2 hHCF(7-34)NH₂ |
| [Ser¹⁰,Leu¹¹,D-Trp¹²] | 4.0 ± 0.7 |
| hHCF(7-34)NH₂ | |

### Example 6

HCF analogues were assayed for their ability to inhibit 1¹²⁵-labeles PTH binding to plasma membranes derived from human osteosaroma cell (B10) in a manner similar to that described in Example 2.

| Structure | Binding K_{I}(nM) |
|---|---|
| hHCF(7-34)NH₂ | 60 |
| [Leu¹¹, [D-Trp¹²]hHCF(7-34)NH₂ | 10 |
| [Asn¹⁰,Leu¹¹,D-Trp¹²]hHCF(7-34)NH₂ | |

## Claims

1. A peptide selected from the group consisting of hHCF(14-34)NH₂, hHCF(13-34)NH₂, hHCF(12-34)NH₂, hHCF(11-34)NH₂, hHCF(10-34)NH₂, hHCF(9-34)NH₂ and hHCF(8-34)NH₂.

2. A peptide of Claim 1 wherein (a) Ala³⁴ is replaced with Tyr³⁴, and/or (b) Phe²³ is replaced with an amino acid selected from the group consisting of the D- or L-stereoisomers of Leu, Nle, Val, Tyr, Trp, beta-naphthylalanine and alpha-naphthylalanine and/or (c) any or each of Asp¹⁰, Lys¹¹ or Ile¹⁴ is replaced with an N-alkyl amino acid or a D- or L-stereoisomer of an amino acid, in particular Asn, Leu and His.

3. A peptide selected from the group consisting of hHCF(12-34)NH₂, hHCF(11-34)NH₂, hHCF(10-34)NH₂, hHCF(9-34)NH₂ and hHCF(8-34)NH₂ wherein Gly¹² is replaced with an amino acid selected from the group consisting of the D- or L-stereoisomers of Trp, Pro, Ala, Aib, naphthyl Ala, alpha-MeTrp and NMeGly.

4. A peptide of Claim 3 wherein (a) Ala³⁴ is replaced with Tyr³⁴, and/or (b) Phe²³ is replaced with an amino acid selected from the group consisting of the D- or L-stereoisomers of Leu, Nle, Val, Tyr, Trp, beta-naphthylalanine and alpha-napthylalanine, and/or (c) any or each of Asp¹⁰, Lys¹¹ or Ile¹⁴ is replaced with an N-alkyl amino acid or a D- or L-stereoisomer of an amino acid, in particular Asn, Leu or His.

5. A peptide which is [Nle^{8,18}] hHCF(8-34) NH₂ wherein (a) Ala³⁴ is replaced with Tyr³⁴, and/or (b) Phe²³ is replaced with an amino acid selected from the group consisting of the D- or L-stereoisomers of Leu, Nle, Val, Tyr, Trp, beta-naphthylalanine and alpha-naphthylalanine, and/or (c) any or each of Asp¹⁰, Lys¹¹ or Ile¹⁴ is replaced with an N-alkylamino acid or a D- or L-stereoisomer of an amino acid, in particular Asn, Leu or His, and/or (d) Gly¹² is replaced with an amino acid selected from the group consisting of D- or L- stereoisomers of Trp, Pro, Ala, Aib, naphthyl Ala, alpha-MeTrp and NMeGly.

6. A peptide of Claim 1 which is hHCF(14-34)NH₂.

7. A peptide of Claim 3 which is [D-Trp¹²]hHCF(10-34)NH₂.

8. A peptide selected from the group consisting of
hHCF(3-34)NH₂, [Tyr³⁴]hHCF(3-34)NH₂,
[Nle^{8,18}, Tyr³⁴]hHCF(3-34)NH₂,
[Nle^{8,18}]hHCF(3-34)NH₂; hHCF(4-34)NH₂;
[Tyr³⁴]hHCF(4-34)NH₂, [Nle^{8,18},
Tyr³⁴]hHCF(4-34)NH₂, [Nle^{8,18}]hHCF(4-34)NH₂;
hHCF(5-34)NH₂, [Tyr³⁴]hHCF(5-34)NH₂,
[Nle^{8,18}, Tyr³⁴]hHCF(5-34)NH₂,
[Nle^{8,18}]hHCF(5-34)NH₂; hHCF(6-34)NH₂,
[Tyr³⁴]hHCF(6-34)NH₂, [Nle^{8,18},
Tyr³⁴]hHCF(6-34)NH₂, [Nle^{8,18}]hHCF(6-34)NH₂;
hHCF(7-34)NH₂, [Tyr³⁴]hHCF(7-34)NH₂,
[Nle^{8,18}, Tyr³⁴]hHCF(7-34)NH₂ and
[Nle^{8,18}]hHCF(7-34)NH₂.

9. A peptide of Claim 8 where (a) Gly¹² is replaced with an amino acid selected from the group consisting of the L- or D- stereoisomers of Trp, Pro, Ala, Aib, naphthyl Ala, alpha-MeTrp and NMeGly, and/or (b) any one or more of Asp¹⁰, Lys¹¹ or Ile¹⁴ is replaced with any N-alkyl amino acid or a D- or L-stereoisomer of an amino acid, in particular Asn, Leu or His.

10. A peptide of Claim 8 or 9 which is
[D-Trp¹²]hHCF(7-34)NH₂, [D-Trp¹²,
Tyr³⁴]hHCF(7-34)NH₂, [D-Trp¹², Nle^{8,18},
Tyr³⁴]hHCF(7-34)NH₂, [D-Trp¹²,
Nle^{8,18}]hHCF(7-34)NH₂, [Asn¹⁰,
Leu¹¹]hHCF(7-34)NH₂,[Asn¹⁰,
D-Trp¹²]hHCF(7-34)NH₂,[Ser¹⁰, Leu¹¹,
D-Trp¹²]hHCF(7-34)NH₂, [Ser¹⁰, Pro¹¹
D-Trp¹²]hHCF(7-34)NH₂, [Ser¹⁰, Sar¹¹
D-Trp¹²]hHCF(7-34)NH₂, [Leu¹¹, D-Trp¹²,
Tyr³⁴]hHCF(7-34)NH₂.

11. The use of a peptide as claimed in any one of claims 1 to 10 for the preparation of a medicament useful for acting upon a HCF receptor in mammals.

12. A pharmaceutical composition which comprises an effective amount of a peptide of any of Claims 1 to 10 and a pharmaceutically acceptable carrier.

## Patentansprüche

1. Peptid, ausgewählt aus der Gruppe, bestehend aus hHCF(14-34)NH₂, hHCF(13-34)NH₂, hHCF(12-34)NH₂, hHCF(11-34)NH₂, hHCF(10-34)NH₂, hHCF(9-34)NH₂ und hHCF(8-34)NH₂.

2. Peptid nach Anspruch 1, worin (a) Ala³⁴ durch Tyr³⁴ ersetzt ist und/oder (b) Phe²³ durch eine Aminosäure ersetzt ist, ausgewählt aus der Gruppe, bestehend aus den D- oder L-Stereoisomeren von Leu, Nle, Val, Tyr, Trp, beta-Naphthylalanin und alpha-Naphthylalanin, und/oder (c) irgendeines oder jedes von Asp¹⁰, Lys¹¹ oder Ile¹⁴ durch eine N-Alkylaminosäure oder ein D- oder L-Stereoisomer einer Aminosäure, insbesondere Asn, Leu und His, ersetzt ist.

3. Peptid, ausgewählt aus der Gruppe, bestehend aus hHCF(12-34)NH₂, hHCF(11-34)NH₂, hHCF(10-34)NH₂, hHCF(9-34)NH₂ und hHCF(8-34)NH₂, worin Gly¹² durch eine Aminosäure ersetzt ist, ausgewählt aus der Gruppe, bestehend aus den D- oder L-Stereoisomeren von Trp, Pro, Ala, Aib, Naphthyl-Ala, alpha-MeTrp und NMeGly.

4. Peptid nach Anspruch 3, worin (a) Ala³⁴ durch Tyr³⁴ ersetzt ist und/oder (b) Phe²³ durch eine Aminosäure ersetzt ist, ausgewählt aus der Gruppe, bestehend aus den D- oder L-Stereoisomeren von Leu, Nle, Val, Tyr, Trp, beta-Naphthylalanin und alpha-Naphthylalanin, und/oder (c) irgendeines oder jedes von Asp¹⁰, Lys¹¹ oder Ile¹⁴ durch eine N-Alkylaminosäure oder ein D- oder L-Stereoisomer einer Aminosäure, insbesondere Asn, Leu und His, ersetzt ist.

5. Peptid, das [Nle^{8,18}]hHCF(8-34)NH₂ ist, worin (a) Ala³⁴ durch Tyr³⁴ ersetzt ist und/oder (b) Phe²³ durch eine Aminosäure ersetzt ist, ausgewählt aus der Gruppe, bestehend aus den D- oder L-Stereoisomeren von Leu, Nle, Val, Tyr, Trp, beta-Naphthylalanin und α-Naphthylalanin, und/oder (c) irgendeines oder jedes von Asp¹⁰, Lys¹¹ oder Ile¹⁴ durch eine N-Alkylaminosäure oder ein D- oder L-Stereoisomer einer Aminosäure, insbesondere Asn, Leu oder His, ersetzt ist, und/oder (d) Gly¹² durch eine Aminosäure ersetzt ist, ausgewählt aus der Gruppe, bestehend aus den D- oder L-Stereoisomeren von Trp, Pro, Ala, Aib, Naphthyl-Ala, alpha-MeTrp und NMeGly.

6. Peptid nach Anspruch 1, das hHCF(14-34)NH₂ ist.

7. Peptid nach Anspruch 3, das [D-Trp¹²]hHCF(10-34)NH₂ ist.

8. Peptid, ausgewählt aus der Gruppe, bestehend aus
hHCF(3-34)NH₂, [Tyr³⁴]hHCF(3-34)NH₂,
[Nle^{8,18}, Tyr³⁴]hHCF(3-34)NH₂,
[Nle^{8,18}]hHCF(3-34)NH₂; hHCF(4-34)NH₂;
[Tyr³⁴]hHCF(4-34)NH₂, [Nle^{8,18},
Tyr³⁴]hHCF(4-34)NH₂, [Nle^{8,18}]hHCF(4-34)NH₂;
hHCF(5-34)NH₂, [Tyr³⁴]hHCF(5-34)NH₂,
[Nle^{8,18}, Tyr³⁴]hHCF(5-34)NH₂,
[Nle^{8,18}]hHCF(5-34)NH₂; hHCF(6-34)NH₂,
[Tyr³⁴]hHCF(6-34)NH₂, [Nle^{8,18},
Tyr³⁴]hHCF(6-34)NH₂, [Nle^{8,18}]hHCF(6-34)NH₂;
hHCF(7-34)NH₂, [Tyr³⁴]hHCF(7-34)NH₂,
[Nle^{8,18}, Tyr³⁴]hHCF(7-34)NH₂ und
[Nle^{8,18}]hHCF(7-34)NH₂.

9. Peptid nach Anspruch 8, worin (a) Gly¹² durch eine Aminosäure ersetzt ist, ausgewählt aus der Gruppe, bestehend aus den L- oder D-Stereoisomeren von Trp, Pro, Ala, Aib, Naphthyl-Ala, alpha-MeTrp und NMeGly, und/oder (b) irgendeines oder mehrere von Asp¹⁰, Lys¹¹ oder Ile¹⁴ durch eine N-Alkylaminosäure oder ein D- oder L-Stereoisomer einer Aminosäure, insbesondere Asn, Leu oder His, ersetzt sind.

10. Peptid nach Anspruch 8 oder 9, das
[D-Trp¹²]hHCF(7-34)NH₂, [D-Trp¹²,
Tyr³⁴]hHCF(7-34)NH₂, [D-Trp¹², Nle^{8,18},
Tyr³⁴]hHCF(7-34)NH₂, [D-Trp¹², Nle^{8,18}]hHCF(7-34)NH₂, [Asn¹⁰,
Leu¹¹]hHCF(7-34)NH₂,[Asn¹⁰,
D-Trp¹²]hHCF(7-34)NH₂,[Ser¹⁰, Leu¹¹,
D-Trp¹²]hHCF(7-34)NH₂, [Ser¹⁰, Pro¹¹,
D-Trp¹²]hHCF(7-34)NH₂, [Ser¹⁰, Sar¹¹
D-Trp¹²]hHCF(7-34)NH₂, [Leu¹¹, D-Trp¹²,
Tyr³⁴]hHCF(7-34)NH₂.
ist.

11. Verwendung eines Peptids nach einem der Ansprüche 1 bis 10 zur Herstellung eines Medikaments, das zur Einwirkung auf einen HCF-Rezeptor bei Säugern geeignet ist.

12. Pharmazeutisches Präparat, das eine wirksame Menge eines Peptids nach einem der Ansprüche 1 bis 10 und einen pharmazeutisch verträglichen Trägerstoff umfaßt.

## Revendications

1. Peptide choisi dans le groupe constitué par hHCF(14-34)NH₂, hHCF(13-34)NH₂, hHCF(12-34)NH₂, hHCF(11-34)NH₂, hHCF(10-34)NH₂, hHCF(9-34)NH₂ et hHCF(8-34)NH₂.

2. Peptide selon la revendication 1, dans lequel (a) Ala³⁴ est remplacé par Tyr³⁴ et/ou (b) Phe²³ est remplacé par un acide aminé choisi dans le groupe constitué par les stéréoisomères D ou L de Leu, Nle, Val, Tyr, Trp, β-naphtylalanine et α-naphtylalanine et/ou (c) l'un quelconque ou chacun de de Asp¹⁰, Lys¹¹ ou Ile¹⁴ est remplacé par un acide N-alkylaminé ou un stéréoisomère D ou L d'un acide aminé, en particulier Asn, Leu et His.

3. Peptide choisi dans le groupe constitué par hHCF(12-34)NH₂, hHCF(11-34)NH₂, hHCF(10-34)NH₂, hHCF(9-34)NH₂ et hHCF(8-34)NH₂, dans lequel Gly¹² est remplacé par un acide aminé choisi dans le groupe constitué par les stéréoisomères D ou L de Trp, Pro, Ala, Aib, naphtyl-Ala, α-MeTrp et NMeGly.

4. Peptide selon la revendication 3, dans lequel (a) Ala³⁴ est remplacé par Tyr³⁴ et/ou (b) Phe²³ est remplacé par un acide aminé choisi dans le groupe constitué par les stéréoisomères D ou L de Leu, Nle, Val, Tyr, Trp, β-naphtylalanine et α-naphtylalanine et/ou (c) l'un quelconque ou chacun de Asp¹⁰, Lys¹¹ ou Ile¹⁴ est remplacé par un acide N-alkylaminé ou un stéréoisomère D ou L d'un acide aminé, en particulier Asn, Leu ou His.

5. Peptide, qui est [Nle^{8,18}] hHCF(8-34)NH₂, dans lequel (a) Ala³⁴ est remplacé par Tyr³⁴ et/ou (b) Phe²³ est remplacé par un acide aminé choisi dans le groupe constitué par les stéréoisomères D ou L de Leu, Nle, Val, Tyr, Trp, β-naphtylalanine et α-naphtylalanine et/ou (c) l'un quelconque ou chacun de Asp¹⁰, Lys¹¹ ou Ile¹⁴ est remplacé par un acide N-alkylaminé ou un stéréoisomère D ou L d'un acide aminé, en particulier Asn, Leu ou His et/ou (d) Gly¹² est remplacé par un acide aminé choisi dans le groupe constitué par les stéréoisomères D ou L de Trp, Pro, Ala, Aib, naphtyl-Ala, α-MeTrp et NMeGly.

6. Peptide selon la revendication 1, qui est hHCF(14-34)NH₂.

7. Peptide selon la revendication 3, qui est [D-Trp¹²]hHCF(10-34)NH₂.

8. Peptide choisi dans le groupe constitué par
hHCF(3-34)NH₂, [Tyr³⁴]hHCF(3-34)NH₂,
[Nle^{8,18}, Tyr³⁴]hHCF(3-34)NH₂,
[Nle^{8,18}]hHCF(3-34)NH₂; hHCF(4-34)NH₂;
[Tyr³⁴]hHCF(4-34)NH₂, [Nle^{8,18},
Tyr³⁴]hHCF(4-34)NH₂, [Nle^{8,18}]hHCF(4-34)NH₂;
hHCF(5-34)NH₂, [Tyr³⁴]hHCF(5-34)NH₂,
[Nle^{8,18}, Tyr³⁴]hHCF(5-34)NH₂,
[Nle^{8,18}]hHCF(5-34)NH₂; hHCF(6-34)NH₂,
[Tyr³⁴]hHCF(6-34)NH₂, [Nle^{8,18},
Tyr³⁴]hHCF(6-34)NH₂, [Nle^{8,18}]hHCF(6-34)NH₂;
hHCF(7-34)NH₂, [Tyr³⁴]hHCF(7-34)NH₂,
[Nle^{8,18}, Tyr³⁴]hHCF(7-34)NH₂ et
[Nle^{8,18}]hHCF(7-34)NH₂.

9. Peptide selon la revendication 8, dans lequel (a) Gly¹² est remplacé par un acide aminé choisi dans le groupe constitué par les stéréoisomères D ou L de Trp, Pro, Ala, Aib, naphtyl-Ala, α-MeTrp et NMeGly et/ou (b) l'un quelconque ou plusieurs de Asp¹⁰, Lys¹¹ ou Ile¹⁴ est remplacé par un acide N-alkylaminé quelconque ou un stéréoisomère D ou L d'un acide aminé, en particulier Asn, Leu et His.

10. Peptide selon la revendication 8 ou 9, qui est
[D-Trp¹²]hHCF(7-34)NH₂, [D-Trp¹²,
Tyr³⁴]hHCF(7-34)NH₂, [D-Trp¹², Nle^{8,18},
Tyr³⁴]hHCF(7-34)NH₂, [D-Trp¹²,
Nle^{8,18}]hHCF(7-34)NH₂, [Asn¹⁰,
Leu¹¹]hHCF(7-34)NH₂,[Asn¹⁰,
D-Trp¹²]hHCF(7-34)NH₂,[Ser¹⁰, Leu¹¹,
D-Trp¹²]hHCF(7-34)NH₂, [Ser¹⁰, Pro¹¹,
D-Trp¹²]hHCF(7-34)NH₂, [Ser¹⁰, Sar¹¹,
D-Trp¹²]hHCF(7-34)NH₂, [Leu¹¹, D-Trp¹²,
Tyr³⁴]hHCF(7-34)NH₂.

11. Utilisation d'un peptide selon l'une quelconque des revendications 1 à 10 pour préparer un médicament utile pour agir sur un récepteur HCF chez les mammifères.

12. Composition pharmaceutique, qui comprend une quantité efficace d'un peptide selon l'une quelconque des revendications 1 à 10 et un véhicule pharmaceutiquement acceptable.
